# EUROPEAN PATENT APPLICATION

(11) **EP 0 529 160 A1**
(43) Date of publication of application: **03.03.1993**
(21) Application number: 91307790.5
(22) Date of filing: 23.08.1991
(51) Int. Cl.: C12N 15/86, C12N 15/12, C07K 15/00, A61K 48/00, A61K 37/02, C12P 21/02

(54) **Gene protein products and methods of cell therapy**

(71) Applicant: THE REGENTS OF THE UNIVERSITY OF CALIFORNIA, Alameda, California 94501 (US)
(72) Inventor: Lee, Wen-Hwa, San Diego, California 92130 (US); Lee, Eva Y. H. P., San Diego, California 92130 (US)
(74) Representative: Spall, Christopher John

(57) **Abstract**

A method that produces substantial quantities of a desired polypeptide, by delivering genetic material into insect cells. For example, cloned genes, or gene fragments or, derivates may be defined, utilizing as appropriate vector, into host cells for high level production of high purity protein in substantial quantities.

## Description

### Technical Field

This invention relates, in general, to methods for producing of gene protein products and to methods of cell therapy for treating cells to suppress tumorigenesis.

This invention relates in general, also, to cell therapy and to methods for treating cells to suppress tumorigenesis.

This invention was made with Government support under Grant No. EY05758 with the National Institute of Health, and the University of California. The Government has certain rights in this invention.

### Background Art

Human and animal tissues have been studied, at great length, and numerous defects have been identified at the cellular level. Many of these defects have a genetic basis and are due, in many cases, to a defective or missing gene. The defect may be the result of a point mutation or other cause, leading to a disruption or abnormal change in the nucleotide sequence within the gene itself. The ultimate result of a malfunctioning gene is, of course, the failure to produce gene protein product or, alternatively, the production of gene protein product which is itself defective.

In the event of the identification of a defective gene, in the human or in an animal, gene therapy may be performed. In this regard, a cloned gene may be delivered to the nucleus of the cell to be treated for the purpose of rectifying the abnormal or defective genetic material. The material utilized in this process material is expensive to produce, requiring sophisticated laboratory equipment and the practice of sophisticated molecular genetic techniques. Such techniques are not generally available and since they are, for the most part, confined to a relatively small number of highly sophisticated molecular genetic laboratories.

Because of the cost and general unavailability of gene therapeutic methods, alternative forms of cell therapy are desirable. Analysis of the molecular structure and function of the protein product enables conclusions to be drawn as to the health of the gene producing the product. Frequently, gene protein products can be used for evaluation of interaction among genes for, as an example, the determination of the tumor suppression mechanisms of the body. Regarding tumor suppression mechanisms, reference may be made to the foregoing parent patent applications. In order to facilitate the elucidation of gene function and interaction, it would be highly desirable to have methods for producing gene protein products which were reliable, inexpensive, and which could provide large volumes of the protein in a reliable and predictable manner.

In addition to the value of the gene protein product in the elucidation of genetic function and interaction, the protein itself can be used therapeutically for treatment of defective genetic conditions. In such cases, it would be convenient and effective to introduce into a cell, having defective genetic material, the appropriate gene protein product. Delivery of the protein product would, in some cases, be less expensive and more easily accomplished than the therapeutic administration of the genetic material itself.

For protein therapy, reference may be made to the foregoing mentioned related patent application filed contemporaneously herewith.

As a result of an appreciation of the importance of gene protein products, it would be highly advantageous to have available a technique for preparing and isolating gene protein products in substantially purified form. The availability of intact and biochemically active protein in large quantities, would represent a significant advance for studying the biochemical properties and molecular behavior involved in genetic mechanisms, as well as for therapeutic applications.

In general, for laboratory purposes as distinct from large scale production, gene protein products have been procured from cells, as well as by synthetic production thereof. With regard to derivation from cells, cellular proteins exist only in very small quantities. As a result, it is not practical to attempt to derive sufficiently large quantities of the protein from natural sources.

With regard to synthetic methods of production, attempts to express protein by introducing the coding sequence of a gene into a bacterial expression vector, have only been partially successful. Bacterially produced proteins have poor solubility. Another drawback of using a bacterial expression system is that bacterial cells are unable to modify eukaryotic proteins, and analysis of such proteins could be misleading, if post-translational modifications are required for the normal function of the protein. In summary, bacterially produced proteins generally have poor solubility and may be molecularly defective, thereby limiting their value.

Conventional laboratory techniques for making protein products have suffered from an inability to produce sufficiently large quantities, but also the resulting products have not been sufficiently pure, on a consistent basis. As a representative example of the difficulty in the production of some protein products, TrpE-RB fusion proteins have been developed and a T7 RNA polymerase expression has been utilized, expressing in E.coli, for production of the polypeptide. These methods have proven to be relatively complicated, requiring the practice of sophisticated biochemical techniques. In addition, such methods have serious limitations, since they are capable of producing only very small amounts of the desired polypeptide. In addition, the polypeptides produced by such methods are often not molecularly suitable, as for example, not being phosphorylated.

Therefore, in view of the importance of the gene product polypeptides, it would be highly desirable to have a method for producing such polypeptides, in substantial quantities, having desired biochemical and biophysical characteristics.

Significant progress has been made in understanding the function of genes in maintenance of the health of the organism. As a general rule, it may be stated that the failure of a cellular gene to produce an appropriate protein is the cause of numerous pathologies in the organism. The failure may be due to the fact that an entire gene is missing or because the gene is itself defective for various reasons. In recognition of these factors, significant advances have been made in gene therapy.

For example, Wilms tumor, a childhood cancer of the kidney, is thought to arise by inactivation of a gene on chromosome 11. Using the technique of microcell fusion-mediated transfer of single chromosomes, it has been demonstrated that introduction of a normal chromosome 11 into Wilm's tumor cells suppressed the tumorigenicity. On the other hand, the introduction of chromosomes X and 13 did not have this effect.

While the transfer of entire human chromosomes may have some value, on an experimental basis, it is not feasible for such transfer to be considered for the treatment of genetic defects. For one thing, preparation of suitable chromosomes for therapeutic applications is very exacting, time consuming and expensive. As a result, such a technique has not been found to be acceptable for many applications.

The next logical consideration, given the undesirability of attempting therapeutic use of entire chromosomes, is to deliver to the patient all, or at least operative portions, of the appropriate gene. While such an approach may be more feasible than the delivery of entire chromosomes, gene therapy is desirable for certain applications only.

In this regard, the isolation, sequencing and cloning of the appropriate nucleic acid material is very expensive, and time consuming. In addition, such techniques require a level of sophisticated molecular genetic techniques that are available only in very limited locations in the world. Further, at the present time, such techniques are not adapted for production of large amounts of materials suitable for therapeutic applications.

In view of the above, it would be highly desirable to have a method for specific therapeutic treatment, at the cellular level, utilizing biotechnical techniques, and employing materials which are relatively low-cost, reliable, more generally available and specific in their biochemical action. Further, it would be highly desirable to have methods of treatment which would be capable of permitting delivery of a therapeutic product at the cellular level to effect changes such as the suppression or suppression of tumors. Of course, it would be highly desirable to have a product which could be made, in large quantities, in a consistently purified state and which would be readily and effectively deliverable to the defective cell.

### Disclosure of Invention

It is a primary object of this invention to provide generally safe and specific therapeutic methods and products useful for controlling cancer suppression.

It is a further object of this invention to provide products and methods for controlling cancer suppression which are specific for suppression and eradication of a cancer tumor and which utilize biotechnical methods and products. It is a still further object of the present invention to provide a pharmaceutical composition for therapeutically treating cancer wherein the composition is functional at the cellular and intracellular levels.

It is still another object of this invention to provide a pharmaceutical composition for treating conditions caused by defective, mutant or absence cancer suppressor genes wherein the active ingredient of the composition is a natural or synthetically produced product.

The present invention comprises a method for cell therapy wherein a specific cancer suppressor gene protein product is delivered to the affected cell to accomplish tumor suppression.

The present invention provides a method for treating cancer which reduces the need for conventional radiation and chemotherapy. In addition, the inventive technique may be employed at a very early stage, after a genetic predisposition to cancer has been discovered, but before the onset of tumorigenesis.

A significant advantage of the present invention is that it uses a cancer suppressor gene protein product in a convenient, and relatively inexpensive manner to accomplish cancer suppression at the cellular level.

While there is some uncertainty as to whether inactivation of one or more cancer suppressing genes in a cell is sufficient to cause cancer, the cellular introduction of the gene protein product is a novel and advantageous approach to the treatment of malignancy. A further advantage of the present invention is that, unlike conventional, cytotoxic cancer therapies, the cell therapy herein disclosed accomplishes beneficial changes at the cellular levels, while minimizing trauma to the organism.

It is a primary object of this invention to provide methods for producing substantial amounts of intact and active gene product polypeptides.

It is a further object of this invention to provide methods for the production of specific gene product polypeptides which are identical in structure and function to naturally occurring polypeptides.

Briefly, the above and further objects of the present invention are realized by providing a method for producing substantial quantities of a desired polypeptide, by delivering genetic material into insect cells. For example, cloned genes, or gene fragments or, derivatives may be delivered, utilizing an appropriate vector, into host cells for high level production of high purity protein in substantial quantities.

A significant advantage of the present invention is that it provides a technique for producing substantial quantities of high quality polypeptides for investigation of gene function, at the cellular level.

Another advantage of the present invention is that it provides a technique for such production in a convenient, reliable and repetitive manner, at relatively low cost.

A further advantage of the present invention is that it provides substantial quantities of high quality polypeptides for elucidation of interactions among genes at the cellular and subcellular levels.

### Brief Description of Drawings

The above mentioned and other objects and features of this invention and the manner of attaining them will become apparent, and the invention itself will be best understood by reference to the following description of the embodiment of the invention in conjunction with the accompanying drawings, wherein:
FIG. 1 is a diagrammatic representation of the construction of the baculovirus expression vector for pp110^{RB} synthesis;
FIG. 2A is a Western blot of ppRB infected insect cells;
FIG. 2B is a Western blot identifying cellular extracts from infected cells at up to 72 hours post-infection;
FIG. 3A is a photomicrograph depicting intracellular localization of RB protein;
FIG. 3B is a photomicrograph of infected Sf9 cells;
FIG. 4 is an autoradiograph depicting phosphorylation of RB protein in insect cells and the results of dephosphorylation analysis;
FIG. 5 depicts the electrophoretic analyses of crude lysates, infected Sf9 cells and eluates from pMG3-245 anti-RB;
FIG. 6A depicts a Southwestern DNA binding assay of fusion proteins and baculovirus-expressed pp110^{RB} applied to 10% SDS-PAGE, Coomassie brilliant blue staining;
FIG. 6B is an autoradiograph of a blot from a parallel gel to the gel used to produce FIG. 6A, the blot having been incubated with ³²P-labeled DNA fragments;
FIG. 7 is a chromatogram showing complex formation of baculovirus-expressed RB protein with SV40 T antigen; and
FIG. 8 is a photograph depicting nuclear translocation of purified RB protein after microinjection into the cytoplasm of Saos-2 cells.

### Best Mode for Carrying out the Invention

All references cited in this Application are hereby incorporated by reference and made part of this application. The detailed description is arranged according to the following outline:
A. GENERAL DESCRIPTION.
B. RB GENE PRODUCT EXAMPLE.
   B1. THE CONSTRUCTION OF RECOMBINANT BACULOVIRUS.
   B2. EXPRESSION OF EXOGENOUS RB PROTEIN IN INFECTED INSECT CELLS.
   B3. NUCLEAR LOCALIZATION AND POST-TRANSLATIONAL PHOSPHORYLATION OF EXOGENOUS RB PROTEIN.
   B4. PURIFICATION OF RB PROTEIN FROM INFECTED INSECT CELLS.
   B5. DNA-BINDING ACTIVITY AND SPECIFIC COMPLEX FORMATION WITH SV40 T ANTIGEN.
   B6. NUCLEAR TRANSLOCATION OF PURIFIED RB PROTEIN.
   B7. SUMMARY

### A. GENERAL DESCRIPTION

A method has been invented for the production of gene protein, by the delivery of genetic material to insect cell cultures for production, by the culture, of a specific gene protein product. Utilizing the present invention, high titers of substantially purified, intact and biochemically active proteins have been produced.

The expression system of the present invention has broad applications. Thus, for example, cloned human and animal genes, and fragments, homologs, portions derivatives and portions thereof, may be utilized for the production of the desired protein product. The protein product thus produced, of course, has utility in treatment of defective cells and in the elucidation of gene functions, as the genes interact with one another at the cellular level.

In producing the gene protein product, it has been found that insect cell cultures, since they are eukaryotic in character, are suitable. Conventional vectors, such as viral vectors, may be used for delivery of the genetic material to the cell culture.

For example, viral vectors of insect cell culture have been utilized for the production of the RB gene protein product. In this regard, the baculovirus, Autographa californica nuclear polyhedrosis virus (AcNPV), is utilized as a helper-independent viral expression vector for the high-level production of recombinant proteins in cultured insect cells. The virus is propagated in cultured Fall Army worm Spodoptera frugiperda (Sf9) cells. The virus has a strong temporally regulated promotor of the polyhedrin gene, whose product represents 50% or more of total cellular proteins during a lytic infection. By in vivo recombination, the coding sequence of a foreign gene is placed under the transcriptional control of the polyhedrin promoter, resulting in a high level of protein expression. In addition, the proteins so produced are correctly folded and contain appropriate post-translational modifications similar to those proteins in the original higher eukaryotes.

The present invention is a method of cell therapy wherein a specific cancer suppressor gene protein product is delivered to the affected cell to accomplish tumor suppression. A given cell may be defective in that it has a missing or defective gene thereby leading to a deficiency in protein expression in the cell.

The inventive method relates to using a gene protein product related to the defective gene. The purified protein product is delivered to the affected cell to accomplish, for example, tumor suppression. The product is delivered in a pharmacologically suitable carrier, thereby enabling the protein product to function at the cellular, or subcellular level.

As an example of such a method, an RB gene protein has been delivered to cells having a missing or defective RB gene, which is a cancer suppressor gene. For more information regarding the RB gene, reference may be made to the parent patent applications referred to herein.

In the preferred example, retinoblastoma, a rare childhood cancer of the developing retina, is the prototypic model for studies of recessive oncogenesis. Based on the localization of the involved genetic element to chromosome 13q14, and evidence of its recessive nature, the putative cancer suppressor gene, retinoblastoma susceptibility gene (RB), was cloned. This gene contains 27 exons dispersed within 200 kilobases of genomic DNA and expresses a 4.7 kilobase mRNA transcript in all normal tissues examined. Sequence analysis of the complementary DNA clones revealed a long open reading frame that could encode a hypothetical protein of 928 amino acids. Using antibodies raised against selected epitopes predicted from the RB cDNA sequence, the RB gene product has been identified as a nuclear phosphoprotein with relative molecular mass (Mr) of 110,000-114,000, and was named pp110^{RB}.

In addition to retinoblastoma, the loss of RB gene function has also been implicated in the development of several other tumor types, including breast cancer, osteosarcoma, prostate cancer and small cell lung carcinoma. The recent demonstration that the reintroduction of RB gene, via retrovirus-mediated gene transfer, into retinoblastoma, osteosarcoma or prostate carcinoma cells apparently suppresses several aspects of their neoplastic phenotype, including tumorigenicity in nude mice, provides direct evidence for the tumor suppression function of the RB gene. However, the molecular basis of this biological activity has not been defined to date.

Until the present time, elucidation of the biochemical properties and biological functions of cancer suppressor gene products has been hampered by difficulty in obtaining sufficient quantities of such protein because of its low abundance in cells. In this regard, reference may be made to the patent application entitled "Method for Producing Gene Protein Products" which has been filed contemporaneously with this application.

A cell therapy method has been invented for the delivery of specific gene protein product to cells having defective or absent genes. Utilizing the present invention, appropriate amounts of substantially purified, intact and biochemically active gene product proteins can be delivered to defective cells in therapeutically effective dosages.

The cell therapeutic methods of the present invention has broad applications. The protein product has utility, not only in the treatment of defective cells but in the elucidation of gene functions as the genes interact with one another at the cellular level.

A specific example is provided, wherein the retinoblastoma gene protein product, pp110^{RB}, has broad application for treatment of euikaryotic cells having a defective, or missing, RB gene.

It was found that the purified protein can bind DNA and form a specific complex with SV40 T antigen in the same way as the authentic human pp110^{RB}. The prompt nuclear translocation of the protein after microinjection further suggests the active nature, and therapeutic applications for the purified gene product protein.

### B. RB GENE PRODUCT EXAMPLE

The following is a more detailed description of the inventive method for the production of the pp110^{RB} protein. For additional information relating to such protein, as well as the retinoblastoma gene, reference may be made to the following parent patent applications.

Elucidation of the biochemical properties and biological functions of cancer suppressor gene products, such as the RB gene, has been hampered by difficulty in obtaining sufficient quantities of purified protein. This is due, in part, because of its low abundance in cells and, in addition, because attempts to express protein by introducing the coding sequence of the gene into a bacterial expression vector have only been partially successful. Based on these considerations, it was concluded that the problems presented by current technique could be circumvented by expressing a cloned gene in an eukaryotic system. While the specific example of the present invention relates to production of the RB gene protein product, the present invention has utility for, and is related to, the production of the protein products of other eukaryotic genes, including, but not limited to, the cancer suppressor genes.

The baculovirus Autographa californica nuclear polyhedrosis virus (AcNPV) is known to be suitable as a helper-independent viral expression vector for the high-level production of recombinant proteins in cultured insect cells. This virus propagates in cultured Fall Army worn Spodoptera frugiperda (Sf9) cells and has a strong temporally regulated promotor of the polyhedrin gene, whose product represents 50% or more of total cellular proteins during a lytic infection. By in vivo recombination, the coding sequence of a foreign gene can be placed under the transcriptional control of the polyhedrin promoter, resulting in a high level of protein expression. In addition, such proteins may be correctly folded and contain appropriate post-translational modifications like those proteins in the original higher eukaryotes.

To test the feasibility of expressing functional RB protein by the baculovirus system, cloned human RB cDNA, containing the complete coding sequence of the RB gene, was introduced into the AcNPV expression vector and the recombinant viruses were propagated in insect cells. Successful expression of human pp110^{RB}, at high level, by the host-vector system was achieved. The protein produced is phosphorylated and correctly targeted to the nuclei of infected cells. In addition, methods for the purification of RB protein were developed. It was found that the purified protein can bind DNA and form a specific complex with SV40 T antigen in the same way as the authentic human pp110^{RB}. The prompt nuclear translocation of the protein after microinjection further suggests the active nature of the purified RB protein.

### B1. THE CONSTRUCTION OF RECOMBINANT BACULOVIRUS.

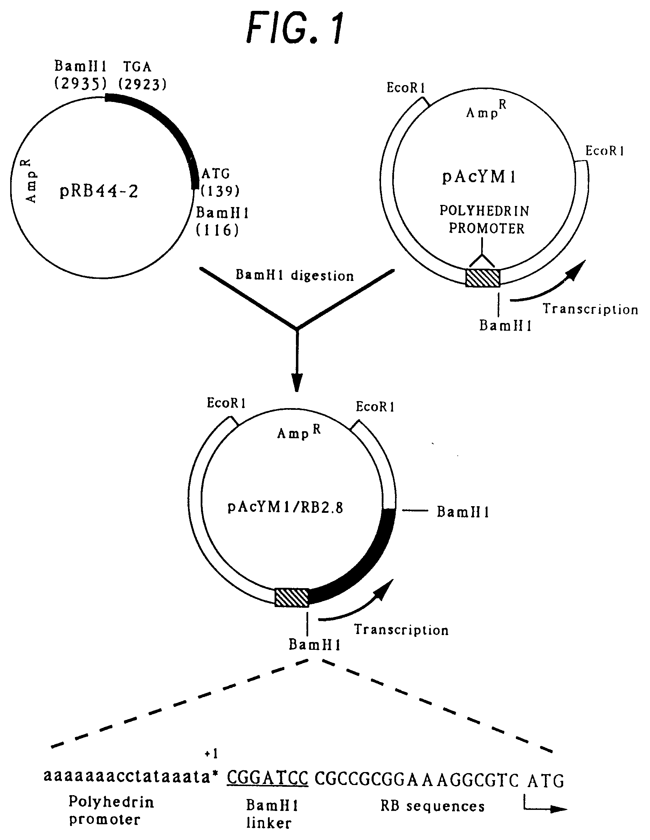

In order to achieve maximal production of the RB protein in the baculovirus expression system, recombinant transfer vectors were constructed with deletion of most of the 5' non-coding sequence of the RB gene. By site-specific mutagenesis, two BamH1 sites were introduced into the RB cDNA at nucleotides 116 and 2935 to facilitate construction of the recombinant transfer vector. As shown in FIG. 1, the resulting pAcYM1/RB2.8 encodes mRNA that contains the entire (60 base pairs) polyhedrin 5' non-coding sequence fused to 23 base pairs of the 5' untranslated region of the RB cDNA, followed by the complete coding sequence. This recombinant gene contains no ATG codons upstream of the authentic RB initiation site at nucleotide 139. Thus, the recombinant gene encodes a non-fusion, full-length RB protein.

Referring now to FIG. 1, there is depicted the transfer vector pAcYM1, which has all the upstream sequences of the polyhedrin gene, including the A of the initiating ATG codon, followed by a unique BamH1 site. The transfer vector has been described by Matsuura et al. J. Gen. Virol., 68: 1233-1250, (1987). pRB44-2 contains the complete RB cDNA coding sequence from nucleotides 116 to 2935 subcloned into the BamH1 site of plasmic pGEM1 (Promega). The recombinant baculovirus vector, pAcYM1/RB2.8, was constructed by inserting the 2.8 kb BamH1 fragment from pRB44-2 into the BamH1 site of pAcYM1 in a proper orientation so that the transcription of the RB gene would be under the direct control of the polyhedrin promoter.

In the construction of the baculovirus expression vector for pp110^{RB} synthesis, the following matters were considered. p^{RB}44-2 consists of the complete RB cDNA coding sequence from nucleotide 116 to 2935 subcloned into the BamH1 site of pGEM1. pAcYM1 contains the approximately 7 kb EcoR1 fragment of the viral DNA sequence flanking the polyhedrin gene in which the leader sequence remains intact, but all of the polyhedrin coding sequences except the first A of the ATG are replaced by a BamH1 linker. The recombinant baculovirus vector, pAcYM1/RB2.8, containing polyhedrin promoter-RB cDNA fusion, was constructed by inserting the RB 2.8 kb BamH1 fragment into the BamH1 site of pAcYM1 so that the transcription of the RB gene would be under the direct control of the polyhedrin promoter. The sequence at the junction of the fusion is shown at the bottom of FIG. 1 with the lower case symbol representing the polyhedrin promoter, and the upper case representing the RB cDNA sequence, while the BamH1 linker is underlined. The translation of the fusion gene utilizing the ATG of the RB (nucleotide 139) is indicated by the arrow, whereas a* (+1) of FIG. 1 represents the first A of the translation start codon ATG of the polyhedrin gene.

Transfer of RB cDNA from the recombinant plasmid to the viral genome was achieved by contransfecting pAcYM1/RB2.8 DNA with wild-type Autographa californica nuclear polyhedrosis virus DNA by lipofection (BRL). The recombinant viruses, in which the polyhedrin gene had been inactivated by allelic replacement with the RB gene through homologous recombination, were identified by their distinct plaque morphology as they showed no polyhedrin occlusion bodies in infected cells. The viruses were subjected to three rounds of plaque purification to obtain a pure stock of RB-containing baculovirus, which was designated as AcNPV-Y4 RB.

### B2. EXPRESSION OF EXOGENOUS RB PROTEIN IN INFECTED INSECT CELLS.

Prior to determining whether the AcNPV polyhedrin promotor could drive the expression of human RB gene in heterologous invertebrate cells, Sf9 cells were prepared. Sf9, a clonal isolate of Spodotera frugiperda IPLB-Sf21-AE In vitro, 13: 213-217, (1977) was grown as monolayer or suspension cultures at 27°C in Grace's insect medium supplemented with 3.33 gm/1 of yeastolate, lactalbumin hydrolysate (GIBCO), and 10% heat-inactivated fetal bovine serum (GIMINI) Bull. 1555, (1987) (Texas Agricultural Experiment Station, College Station, TX). In large-scale preparation of cellular lysates, spinner cultures of Sf9 cells were grown in EX-CELL 400 serum-free defined medium (J.R. Scientific). Molt-4 cells, a human T cell leukemia line, were cultured in suspension in RPMI 1640 supplemented with 20% calf serum. Saos-2 cells, an osteosarcoma cell line, were grown in Dulbecco's modified Eagle's medium supplemented with 7.5% fetal bovine serum.

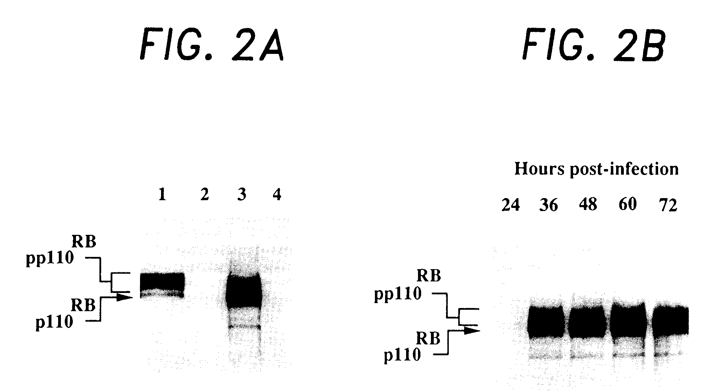

As shown in FIG. 2A, immunoblotting with pMG3-245 monoclonal antibody revealed the appearance of full-length RB protein similar to that of the mammalian cells (lane 1) in extracts of cells infected with AcNPV-Y4 RB (lane 3), but not in the mock or wild-type AcNPV infected cells (lanes 2 and 4). With regard to FIG. 2B, cellular extracts from AcNPV-Y4 RB infected cells were prepared at different times post-infection, in order to determine the optimal timing for RB protein production. The lysates were immunoprecipitated with anti-RB0.47 antibody and immunoblotted with pMG3-245 monoclonal antibody. In FIG. 2B, p110^{RB} and pp110^{RB} represent unphosphorylated and phosphorylated RB proteins, respectively. The production of the RB protein was monitored during the post-infection period to determine the optimal timing for harvesting the cells. As shown in FIG. 2B, RB protein production can be detected at 24 hours after infection and it is significantly increased during the following 12 hours. The level of protein production was maintained through about 72 hours of infection, at which time significant viral lysis of the cells began. To minimize protein degradation associated with cell lysis, infected cells were routinely harvested around 40 hours post-infection.

In detecting the expression of the RB protein, AcNPV-Y4 RB was used to infect Sf9 cells at a MOI of 0.5. At 24, 36, 48, 60 and 72 hours post-infection, 5 x 10⁴ cells were lysed in 1 ml lysis buffer (50 mM Tris-HC1, pH 7.4; 0.2% Nonidet P-40; 1 mM EDTA; 100 mM NaCl; 50 mM NaF and 1 mM PMSF), and the lysates were clarified by centrifugation (4°C, 20,000 x g) for 5 minutes. Lysates were then incubated with anti-RB0.47 antibody, and immunoprecipitates were separated by 7.5% SDS-PAGE. Proteins were then transferred to nitrocellulose paper, following conventional techniques. After overnight blocking, the nitrocellulose paper was incubated with pMG3-245 anti-fRB monoclonal antibody for 3 hours, followed by alkaline phosphatase-conjugated goat anti-mouse IgG and colorigenic substrates, as described in Cell, 54: 275-283, (1988).

### B3. NUCLEAR LOCALIZATION AND POST-TRANSLATIONAL PHOSPHORYLATION OF EXOGENOUS RB PROTEIN.

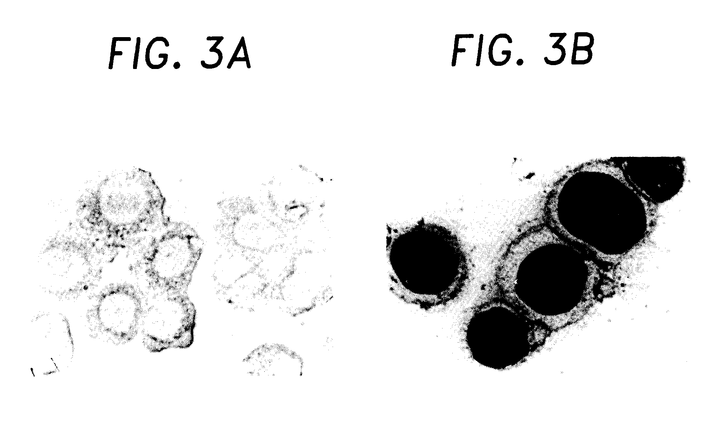

The RB gene encodes a nuclear phosphoprotein of Mr 110,000. To determine whether RB protein produced in insect cells with the baculovirus was targeted to the nucleus, AcNPV-Y4 RB-infected Sf9 cells were immunostained with anti-RB0.47 antibody 40 hours after infection. The intracellular localization of RB protein expressed in insect cells by immunostaining is depicted in FIGS. 3A and 3B. FIG. 3A depicts mock-infected Sf9 cells and FIG. 3B depicts AcNPV-Y4 RB-infected Sf9 cells. As shown in FIGS. 3A and 3B, the infected cells contained unusually large nuclei. Such a condition is characteristic of the cytopathic effect of baculovirus infection. When mock-infected or wild-type AcNPV-infected Sf9 cells were incubated with anti-RB0.47 antibody, no staining was observed (FIG. 3A). However, intense staining was found exclusively in the nuclei of cells infected with AcNPV-Y4 RB (FIG. 3B). Analysis by SDS-PAGE and Western blotting of nuclear and cytoplasmic extracts from AcNPV-Y4 RB infected Sf9 cells confirmed that the exogenous RB protein is present predominantly in the nuclear fraction.

In performing the immunostaining analysis, the following steps were performed. After 40 hours of either mock, wild-type AcNPV, or AcNPV-Y4 infection, Sf9 cells were seeded on poly-L-lysine (Sigma) coated chamber slides (Miles Scientific) and incubated overnight. Slides were washed with phosphate-buffered saline between each of the following steps: cells were first fixed with 4% formaldehyde in 0.04 M phosphate buffer (pH 7.4) for 20 minutes or with acetone (-20°C) for 10 minutes, and immersed in 1% H₂O₂ in methanol for 10 minutes. Fixed cells were preincubated with 2% normal goat serum in PBS for 10 minutes and then incubated overnight with rabbit anti-RB0.47 antibody diluted in 0.02% Triton X-100. After washing, biotinylated goat anti-rabbit IgG (TAGO, Burlingame, CA) was added. One hour later, cells were incubated with AB complex conjugated with horseradish peroxidase (Vector Labs, Burlingame, CA) for 45 minutes and then incubated with substrate. The substrate comprised 0.05% 3,3'-diaminobenzidine tetrahydrochloride and 0.01% H₂O₂ in 0.05 M Tris-HC1, pH 7.6 (Sigma). Reactions were stopped 3 to 5 minutes later by washing cells with PBS. Subsequently, the cells were photographed with a Nikon diaphotomicroscope.

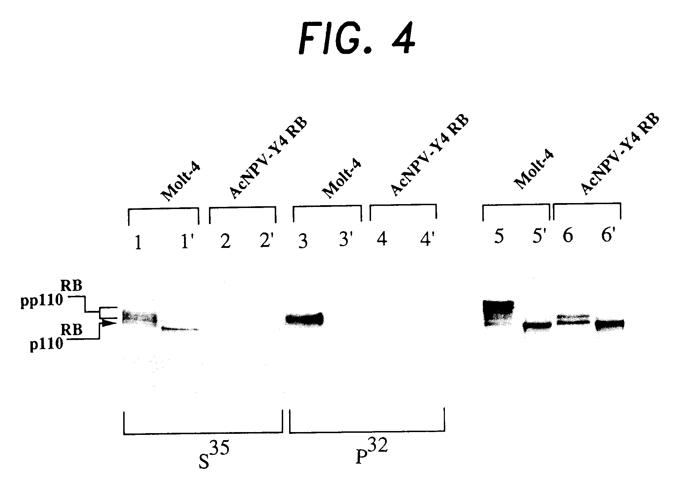

Referring now to FIG. 4, there is shown the results of phosphorylation of RB protein produced in insect cells and dephosphorylation analysis. Forty hours after infection with AcNPV-Y4 RB, Sf9 cells were metabolically labelled with ³⁵S-methionine or ³²P-orthophosphate for 3 hours. Molt-4 was included as the control and cellular lysates were then immunoprecipitated with anti-RB0.47 antibody. The ³⁵S- and ³²P-labeled RB protein immune complexes were separated by SDS-PAGE before (lanes 1, 2, 3 and 4) or after treatment with potato acid phosphatase (PAP) (lanes 1', 2', 3' and 4') and analyzed by autoradiography. Similar dephosphorylation experiments using lysates from unlabeled cells were performed and subjected to Western blot analysis before and after treatment with potato acid phosphatase (lanes 5, 6 and 5', 6' respectively).

With further reference to FIG. 4, phosphorylation of RB protein occurs at multiple serine and threonine residues and accounts for the molecular weight heterogeneity of RB protein in the SDS-PAGE Oncogene Res., 1: 205-214, (1989) Cell, 56: 57-65, (1989). To determine whether RB protein produced in the insect cells undergoes phosphorylation post-translationally, AcNPV-Y4 RB-infected Sf9 cells were metabolically labeled with ³⁵S-methionine or ³²P-orthophosphate for 3 hours at 40 hours after infection. Cell extracts were subjected to immunoprecipitation and analyzed by SDS-PAGE followed by autoradiography. In this regard, please see FIG. 4, lanes 2 and 4, respectively. In parallel, immunoprecipitable RB protein from the same extracts was treated with potato acid phosphatase (PAP) to test the effect of dephosphorylation on RB protein mobility in SDS-PAGE. After dephosphorylation, the ³⁵S-labeled RB protein was reduced from a doublet to a single band of Mr 110,000, (FIG. 4, lane 2'), and radioactivity was almost completely released from ³²P-labeled RB protein (FIG. 4, lane 4'). Dephosphorylation analysis by Western blotting of lysates from unlabeled cells infected with AcNPV-Y4 RB also showed the same band reduction pattern after PAP treatment (FIG. 4, lanes 6 and 6'). These observations indicated that RB protein produced in insect cells was phosphorylated, and the modification also accounted for the molecular weight heterogeneity of this RB protein observed in the SDS-PAGE.

In performing the radiolabeling of Sf9 insect cells and dephosphorylation analysis, the following steps were performed. At 40 hours post-infection, Sf9 cells (3 x 10⁶) in 60 mm dishes were incubated with DME medium lacking either methionine or phosphate and supplemented with 10% fetal calf serum for 30 minutes. The cells were then metabolically radiolabeled by supplementing with 0.25 mCi/ml ³⁵S-methionine (1134 Ci/mmole, NEN) or with 0.25 mCi/ml ³²P-orthophosphate (carrier-free, ICN) for 3 hours. Cell extracts were then prepared in lysis buffer (50 mM Tris-HC1, pH 7.4; 0.2% Nonidet P-40; 1mM EDTA; 100 mM NaCl; 50 mM NaF and 1mM PMSF), and immunoprecipitation with anti-RB0.47 antibody was performed.

Two-thirds of the immunoprecipitated RB protein, from ³⁵S or ³²P-labeled as well as unlabeled cell lysates, were subjected to potato acid phosphatase (PAP, Boehringer) dephosphorylation analysis Oncogene Res., 1: 205-214, (1989). Immune complexes containing the RB protein were incubated with 1.5 units of PAP in reaction buffer (20 mM MES, pH 5.5; 100 mM NaCl; 1 mM MgCl₂; 50 µM leupeptin) for 60 minutes at 37°C. After the reaction, RB protein was analyzed by 7.5% SDS-PAGE, followed by either autoradiography or Western blotting.

### B4. PURIFICATION OF RB PROTEIN FROM INFECTED INSECT CELLS.

Sf9 cells were infected with AcNPV-Y4 RB at a multiplicity of infection (MOI) of 1.0, and forty hours after infection cellular lysates were prepared. Under this condition the total level of RB protein expressed in the baculovirus system was approximately 17-18 mg per liter of infected insect cell culture (⁻10⁹ cells). In this regard, reference may be made to Table 1.

**Table 1**

| Purification of recombinant RB protein from baculovirus infected insect cells. | | | | | |
|---|---|---|---|---|---|
| Step | Total protein (mg) | RB protein (mg) | Yield (%) | Purification fold | Purity (%) |
| Cellular Extract | 670^{a} | 16^{c} | 90^{c} | 1.0 | 2.3 |
| pMG3-245 Immunoaffinity Column | 13.5^{b} | 12.g^{d} | 72^{c}^{,}^{d} | 41.3 | 95 |

| | | | | | |
|---|---|---|---|---|---|
| a. Protein quantitation by the method of Bradford (Bio-RAD). | | | | | |
| b. Protein quantitation by Micro BCA (PIERCE) and spectrophotometry. | | | | | |
| c. Protein quantitation by Western blot and densitometry | | | | | |
| d. Protein quantitation by Coomassie brilliant blue staining and densitometry. | | | | | |

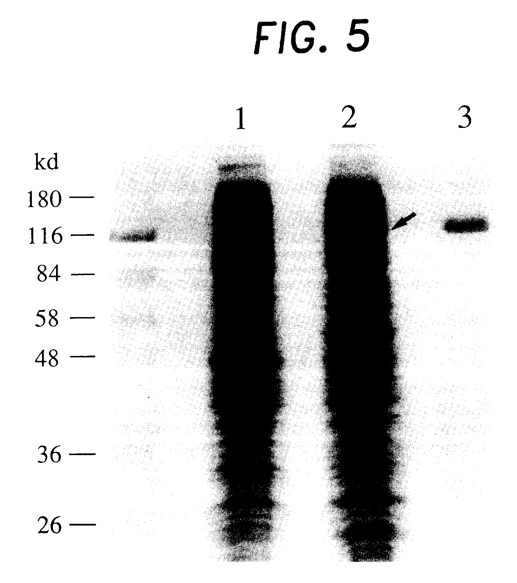

As shown in Table 1, 90% (16 mg) of the RB protein expressed were found in the supernatant after cell disruption, while 10% remained in the insoluble fraction. The RB protein could readily be detected in the cellular lysate (FIG. 5, lane 2) as it represented 2.3% of the total cellular protein. Following the one-step immunoaffinity chromatographic purification, approximately 13.5 mg of proteins could be recovered from the alkaline eluates of the column. To estimate the purity of the eluted RB protein, an aliquot of the eluates corresponding to 2.5 x 10⁵ cells was analyzed by SDS-PAGE and Coomassie brilliant blue staining.

In this regard, reference may be made to FIG. 5 which depicts immunoaffinity chromatographic purification of pp110^{RB}. Crude lysates from 1 x 10⁵, mock- (lane 1) or AcNPV-Y4 RB-infected (lane 2) Sf9 cells as well as an aliquot (corresponding to 2.5 x 10⁵ infected cells) of the eluates from the pMG3-245 anti-RB immunoaffinity chromatography (lane 3) were analyzed by electrophoresis on a 10% SDS-polyacrylamide gel, followed by Coomassie brilliant blue staining. The arrow indicates the position of the RB protein with the expected molecular weight.

As judged by densitometry, the single purification step described herein proved to be efficient, resulting in a preparation of RB protein with 95% purity (FIG. 5, lane 3), a 72% yield and a 41.3-fold of purification (Table 1).

Procedures for the construction of the immunoaffinity column followed the methods described by Schneider et al. and Simanis et al. with minor modification J. Biol. Chem., 257: 10766-10769, (1982) Virology, 144: 88-100, (1985). 2 ml of protein G-Agarose (Genex) were packed in a Bio-Rad column and washed with 0.01 N HC1 followed by the binding buffer (0.1 M sodium acetate, pH 5.0; 0.1 M NaCl). 15 mg of anti-fRB monoclonal antibody (pMG3-245) were applied to the column twice to allow binding. The column was then washed extensively with 0.1 M borate buffer, pH 9.0, and the beads were resuspended in 20 ml of the buffer. Dimethylpimelimidate dihydrochloride (Sigma) was added to a final concentration of 40 mM, and the mixture was agitated for 1 hour at room temperature for the crosslinking reaction to take place. After washing, the remaining reactive groups of the beads were blocked with 40 mM ethanolamine-HC1 in 20 ml of 0.1 M borate buffer, pH 8.0, for 10 minutes at room temperature. The column was then washed with 0.2 M glycine, pH 2.3 and neutralized with Tris buffer (50 mM Tris-HC1, pH 7.4; 100 mM NaCl; 1 mM PMSF; 1 mM EDTA) in which it was stored until required. By measuring OD₂₈₀ of the original monoclonal antibody sample, and that of the flow-through fractions in subsequent steps, it was estimated that approximately 10 mg of pMG3-245 were coupled to the 2 ml of protein G-Agarose beads.

### B5. DNA-BINDING ACTIVITY AND SPECIFIC COMPLEX FORMATION WITH SV40 T ANTIGEN.

To date, two biochemical properties of the RB protein have been described. One is its ability to bind DNA intrinsically Nature, 329: 642-645, (1987), and the other is its ability to form specific complexes with oncoproteins of several DNA tumor viruses Cell, 54: 275-283, (1988); Science, 243: 934-937, (1989); Nature (London), 334: 124-129, (1988). The RB protein purified from baculovirus-infected insect cells was tested for these two known biochemical properties, which have been implicated in the biological functions of the protein.

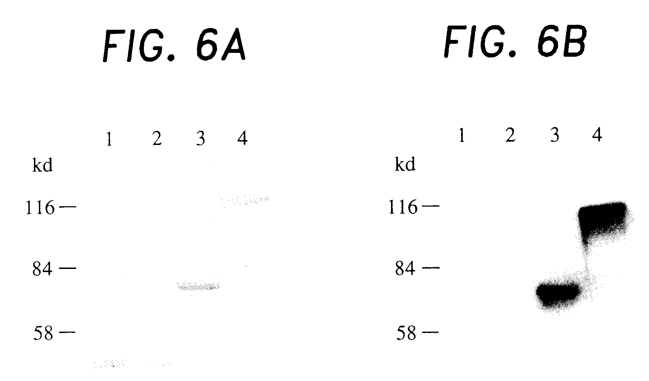

FIGS. 6A and 6B depict Southwestern DNA-binding assays. Six µg of purified trpE-RB fusion proteins, as well as the purified baculovirus-expressed pp110^{RB}, were applied to 10% SDS-PAGE. In the assay depicted in FIG. 6A, Coomassie brilliant blue staining was utilized while in the assay of FIG. 6B, a parallel gel was electrotransferred onto nitrocellulose paper. The blot was then incubated with ³²P-labeled DNA fragments and analyzed by autoradiography. In FIGS. 6A and 6B, the following are shown: Lane 1: RB19-22; lane 2: RB23-27; lane 3: RB19-27; lane 4: purified RB protein from AcNPV-Y4 RB infected insect cells.

With regard to FIGS. 6A and 6B, DNA-binding was assayed by Southwestern analysis in which identical amounts of the trpE-RB fusion proteins, as well as the purified RB protein from insect cells, were separated by 10% SDS-PAGE. The quantity of loaded protein was confirmed by Coomassie brilliant blue staining (FIG. 6A). Another gel run in parallel was electrotransferred to a nitrocellulose membrane, followed by incubation with ³²P-labeled DNA. DNA bound to the protein was then analyzed by autoradiography (FIG. 6B). It has been determined that fusion protein RB19-27, which contains the major domain for interacting with DNA, has a 20-fold higher affinity for DNA than either of two subregions, RB19-22 and RB23-27. In this regard, lane 3 of FIG. 6B can be compared with lanes 1 and 2, while the purified full-length RB protein exhibited a strong DNA-binding activity similar to that of RB 19-27 (FIG. 6B, lane 4). DNA-binding activity of the purified RB protein from insect cells was also demonstrated by retention of the protein by DNA-cellulose and its subsequent elution from the column, at approximately 400 mM NaCl.

In the purification of pp110RB from infected insect cells the following procedures were followed. Sf9 cells were infected with AcNPV-Y4 RB at a MOI of 1.0, and cultured in suspension (1 x 16⁶ cells/ml, 1000 ml). After 40 hours of infection, the cells were pelleted by low-speed centrifugation, washed, and resuspended in an extraction buffer containing 50 mM Tris-HC1, pH 7.4; 0.2% NP-40; 1 mM EDTA; 100 mM NaCl; 10% (v/v) glycerol; 1 mM DTT; 1 mM PMSF; 25 µg/ml leupeptin and 50 units/ml aprotinin. After 15-minute incubation on ice, the sample was clarified by centrifugation (10,000 x g, 4°C for 10 minutes), and the RB-containing supernatant was collected. Immunoaffinity chromatography of pp110^{RB} was carried out on a two-ml-volume column containing anti-fRB monoclonal antibody (pMG3-245) linked to protein G-Agarose as described above. After passing the supernatant through the column four times, the column was washed sequentially with 200 bed-volumes of each of the following: lysis buffer, lysis buffer containing 500 mM NaCl, and washing solution (200 mM NaCl; 1 mM EDTA; 1 mM DTT; 1 mM PMSF; 10% glycerol). Bound proteins were then eluted from the column by alkaline elution buffer containing 20 mM triethylamine, pH 10.8; 200 mM NaCl; 1 mM EDTA; 1 mM DTT; 1 mM PMSF and 10% glycerol. One-ml fractions were collected, immediately neutralized with one-twentieth volume of 1M Tris-HC1 (pH 7.5), and stored at -70°C in 10% glycerol.

In purifying the pp110^{RB} from the infected insect cells, the amount of total protein was determined and, subsequently Southwestern DNA-binding assays and SV40 T antigen binding assays were performed.

The amount of total protein in the elution fraction of the immunoaffinity column was determined by Micro-BCA assay (PIERCE). The eluted protein sample was then analyzed by SDS-PAGE, and the amount of RB protein in the eluates was estimated by Coomassie brilliant blue staining followed by densitometry. The amount of total protein in the cellular extract was measured by the method of Bradford (Bio-Rad) Anal. Biochem., 72: 248-254, (1976). To quantitate RB protein in cellular lysates, Western blotting was performed using serially diluted purified RB protein as standard followed by densitometric comparison of the band intensity. In this regard, reference may be made to Table 1.

Protein blotting was performed, utilizing conventional techniques. Incubation of blots with radiolabeled DNA followed the protocols described by Bowen et al. Nucleic Acids Res. 8: 1-21, (1980). The procedure was carried out at room temperature. Blots were rinsed briefly with water and then washed three times with 6M urea; 0.2% NP-40 (20 min each), followed by four washes (30 min each) with DNA-binding buffer (10 mM Tris-HC1, pH 7.0; 1 mM EDTA; 50 mM NaCl; 0.2% BSA; 0.2% Ficoll 400 and 0.2% polyvinyl pyrolidone). The blots were then incubated for 30 min in DNA-binding buffer containing ³²P-labeled DNA. pGEM1 DNA linearlized by EcoR1 was labeled with α-³²P deoxynucleotides (Amersham, >3000 Ci/mmol) by random priming and was used as the probe. After hybridization, blots were washed three times (10 min each) with DNA-binding buffer, air-dried, and analyzed by autoradiography. TrpE-RB fusion proteins were included as controls. Each trpE-RB fusion protein was named according to the exons of the RB gene that the protein contains. Thus, RB19-22, RB23-27, and RB19-27 spanned the regions of pp110^{RB} from exon 19 to 22 (amino acids 612-775), exon 23 to 27 (amino acid 776-928) and exon 19 to 27 (amino acid 612-928) respectively.

SV40 T antigen was purified by immunoaffinity chromatography from Ad-SV X1-infected 293 cells J. Virol., 53: 1001-1004, (1985); Cold Spring Harbor Press. Cold Spring Harbor, NY pp .187-192, (1982) and anti-T monoclonal PAB419 antibody was obtained from Oncogene Inc. A known complex formation assay was performed, with minor modification, in which 800 ng of baculovirus expressed RB protein was mixed with 1 ml of EBC buffer (50 mM Tris-HC1, pH 8.0, 120 mM NaCl and 0.5% Nonidet P40) containing 1 mM PMSF, 25 µg/ml leupeptin and 50 units/ml aprotinin. 800 ng of purified T was added to the mix and mixture was incubated on ice for 90 minutes. Aliquots of the mixture were immunoprecipitated with either anti-RB0.47 or PAB 419 antibody and subjected to Western blotting analysis. Blots were sequentially reacted with pMG3-245 followed by PAB419. After incubating with alkaline phosphatase-conjugated goat anti-mouse IgG, the blots were developed with colorigenic substrates.

To test the ability of the purified RB protein in forming a specific complex with SV40 T antigen, equal amounts of RB protein and T antigen were mixed, and aliquots of the mixture were immunoprecipitated with either anti-RB0.47 antibody or anti-T antibody PAB419.

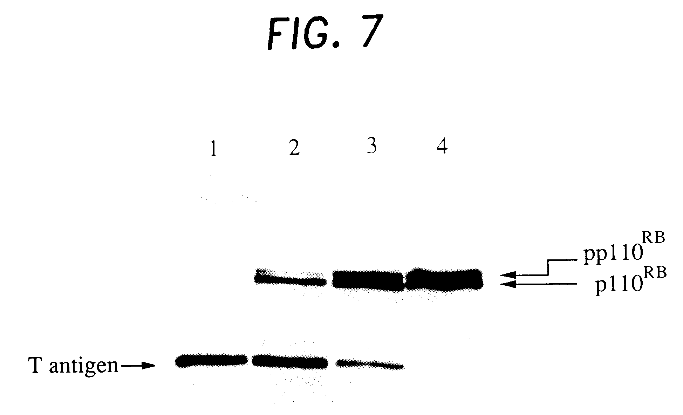

In this regard, FIG. 7 depicts complex formation of baculovirus-expressed RB protein with SV40 T antigen. Purified baculovirus-expressed RB protein were mixed with purified T antigen in vitro. Identical aliquots of the mixtures were then immunoprecipiated with PAB419 (lane 2) or anti-RB0.47 (lane 3) and analyzed by Western blotting. Lanes 1 and 4 show purified SV40 T antigen immunoprecipiated with PAB419, and purified baculovirus-expressed RB protein immunoprecipitated with anti-RB0.47 antibody respectively.

As shown in FIG. 7, mixing of RB protein with T antigen in vitro resulted in the co-immunoprecipitation of RB protein with PAB419 (lane 2), as well as the co-immunoprecipitation of T with anti-RB0.47 antibody (lane 3). These data demonstrated that RB protein from baculovirus-infected insect cells are capable of forming a specific complex with SV40 T antigen.

### B6. NUCLEAR TRANSLOCATION OF PURIFIED RB PROTEIN.

After determining that the purified protein retained the two known biochemical activities of RB in vitro, the behavior of the purified protein in vivo, was investigated. Purified RB protein was injected into the cytoplasm of Saos-2 cells, an osteosarcoma cell line which contains a defective RB gene with deletion of exons 21-27 and encodes a C-terminal truncated RB protein (p95) Proc. Natl. Acad. Sci. U.S.A., 87: 6-10, (1990). This protein is located in the cytoplasm in such minute amounts that it is not recognized by the anti-RB0.47 antibody used herein, in view of the fact that the antibody is directed against the C-terminus of RB protein. Immediately after injection, cells were fixed and subjected to immunostaining analysis.

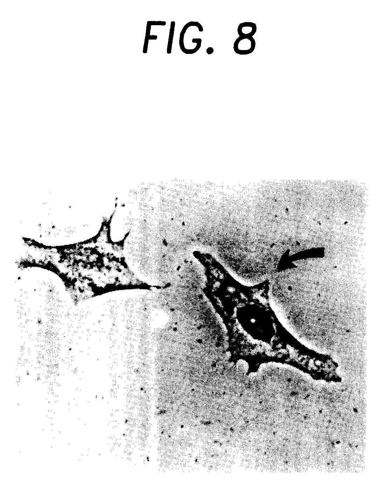

FIG. 8 depicts nuclear translocation of the purified RB protein after microinjection into cytoplasms of Saos-2 cells. The cells were injected with purified RB protein and subjected to immunostaining analysis. The arrow indicates the intense staining of the nucleus after microinjection, as compared to that of uninjected cells.

As shown in FIG. 8, intense staining of the nucleus of the injected cell was found (arrow) as compared to that of the uninjected control, indicating the rapid transport of the injected protein into the nuclei. Since RB protein has been known as a nuclear protein, the prompt and accurate nuclear translocation of purified protein, after microinjection, further suggests that the protein is active in vivo.

For microinjection, purified RB protein was dialyzed into injection buffer containing 20 mM Tris-HC1, pH 7.4; 10 mM KC1; 0.1 mM EDTA; 0.1 mM DTT and 2% glycerol to a final concentration of 0.5 mg/ ml. Saos-2 cells, growing on glass chamber slides were microinjected according to conventional techniques, using glass capillary needles (Eppendorf). An Eppendorf micromanipulator, equipped with a vacuum and pressure device, and an inverted phase-contrast microscope (Nikon) were employed for micromanipulation of the capillary and visualization of the microinjection process. After microinjection, the cells were immediately fixed by 4% formaldehyde in 0.04 M phosphate buffer (pH 7.4) and subjected to immunostaining analysis.

### B7. SUMMARY

As the foregoing has disclosed, it has been demonstrated that the human retinoblastoma gene product can be expressed efficiently under the transcriptional control of the baculoviral polyhedrin promoter. The attempt to express RB protein at high levels has long been regarded as difficult since it was suspected that RB protein might hinder or even be "toxic" to the growth of cells. The transcription of foreign genes from the polyhedrin promoter occurs late in infection, following production of extracellular viral particles and the shut-off of cellular and most viral genes. The baculovirus-insect cell system is therefore advantageous for the synthesis of proteins, such as the RB protein which may be detrimental to cell growth when overproduced. Another advantage of this system is the similarity in protein processing pathways of insect and mammalian cells.

The RB protein produced has been shown to be accurately targeted to the nuclei of insect cells, implying that mammalian nuclear translocation signals are also recognized by insect cells. Although glycosylation of recombinant proteins in the baculovirus expression system seems limited to the O-linked and N-linked oligosaccharides of the high mannose-type, appropriate phosphorylation of foreign proteins has been reported for the expression of c-myc and HTLV-I p40^{x} J. Virol. RB protein has previously been shown to be phosphorylated but not glycosylated, making the baculovirus expression system suitable for the production of functional RB protein.

As disclosed herein, the RB protein produced in infected insect cells is post-translationally phosphorylated, and multiple bands can be differentiated by Western blotting analysis, just as in the case of authentic mammalian RB protein. However, as judged by band intensity, un- and hypophosphorylated forms are predominant when compared to the hyperphosphorylated RB protein. At present, it is not known whether this phenomenon is a reflection of the cell cycle status of the population, during a viral lytic infection, or is simply due to the insufficient phosphorylation of the protein by insect kinases because of the massive amount of exogenous RB present in the cells. Precise mapping of phosphorylation sites in the RB protein will be necessary in order to determine whether the phosphorylation patterns are truly identical to that of mammalian protein.

The total level of recombinant RB protein expressed in the baculovirus system is about 17-18 mg per liter of infected insect cell culture (⁻10⁹ cells). This level of expression is comparable to other mammalian proteins produced by this system, such as 10-20 mg/l for interleukin 2 The Banbury Report. Fields, B., Martin, M. A. and Kamley, D. (ed.), 22: 319-328, (1985) Cold Spring Harbor Laboratory Press, Cold Spring Harbor, and 4-5 mg/l for P210 BCR-ABL Oncogene, 4: 759-766, (1989). The high level of expression of RB protein may be enhanced by using a recombinant transfer vector containing the intact polyhedrin 5' untranslated region, fused with the RB cDNA deprived of most of its 5' non-coding region. This sequence of the RB mRNA is highly G+C rich, a factor which may favor the formation of stable secondary structures. These structures, when present in front of an initiation codon, are thought to decrease the translational efficiency of the corresponding mRNA. Five-to ten-fold enhancement of the in vitro translation of RB mRNA has been demonstrated by the replacement of RB 5' untranslated sequence with that of the alfalfa mosaic virus (AMV) RNA4, or β-globin mRNA, further suggesting the potential adverse effect, on the translation, EMBO J., (in press), (1990) of RB 5'non-coding sequence. The presence of long 5' untranslated sequences of the foreign genes has also been shown to affect the recombinant protein expression in the baculovirus system. Since the polyhedrin promoter is very A+T-rich, it has been concluded that the long and G+C-rich 5' non-coding sequence be trimmed from the RB cDNA, prior to the insertion into the transfer vector, for optimal expression of pp110^{RB}.

Several different protocols for the elution of RB protein from affinity columns have been tested in an attempt to minimize the denaturation of protein during the purification process. Since not much is known of the biological functions and biochemical properties of RB protein, the only two parameters that can be used as measures of the integrity of purified protein are the activities of DNA-binding and complex formation with SV40 T antigen. It was found that the present elution condition, using 20 mM triethylamine at pH 10.8 was effective in preserving the biochemical properties of the protein. Rapid nuclear translocation of the purified protein from the cytoplasm after microinjection further demonstrated that the protein was active under this elution condition. Elution of the protein at extreme pH (200 mM glycine, pH 2.3 or 100 mM triethylamine, pH 11.5) tended to denature the protein in that the aforementioned two activities were greatly diminished. This was also made evident by the formation of insoluble aggregates, after long term storage.

While it has been previously reported that only the unphosphorylated RB protein can bind SV40 T antigen in D2C2 cells, a stable transformant of monkey kidney cell line CV1-P by SV40 T antigen Cell, 56: 57-65, (1989), it was found that certain hypophosphorylated forms of the RB protein were able to form complexes with the SV40 T antigen. This was reproducibly demonstrated with the in vitro mixing of T antigen with purified RB protein from AcNPV-Y4 RB infected-insect cells, or with Molt-4 lysates. The same phenomenon has been observed when Cos cells for in vivo complex formation were used (FIG. 7). Since phosphorylation of the RB protein oscillated during the cell cycle in a phase-specific manner and the complex formation between RB and viral oncoproteins has been implicated in the transforming activity of these DNA tumor viruses, the significance of the association between hypophosphorylated RB protein and SV40 T antigen awaits future elucidation.

The availability of significant amounts of soluble, intact and presumably active RB protein, utilizing the baculovirus-insect cell system represents a major advance for future studies of the biochemical and biophysical properties of the RB gene product. Possible applications include analyses of associated cellular proteins, isolation of the specific DNA sequence with which they interact, and three-dimensional structural studies of the RB protein utilizing X-ray crystallography. The elucidation of the biological function of the retinoblastoma gene in cancer suppression can also be facilitated. The possible involvement of RB in cell growth and differentiation, directly tested by microinjection, are now under active investigation.

Some of the abbreviations used in this specification are: cDNA, complementary DNA; kd, kilodalton; kb, kilobase; SDS, sodium dodecyl sulfate; PAGE, polyacrylamide gel electrophoresis; NP-40, Nonidet P-40; MES, (2-[N-Morpholino]ethanesulfonic acid) sodium salt; MOI, multiplicity of infection; Mr, relative molecular mass; PAP, potato acid phosphatase. The protein product, identified herein as "pp110^{RB}" is the same protein product identified elsewhere as "ppRB¹¹⁰."

While particular embodiments of the present invention have been disclosed, it is to be understood that various different modifications are possible and are contemplated within the true spirit and scope of the appended claims. There is no intention, therefore, of limitations to the exact abstract or disclosure herein presented.

## Claims

1. A method for producing a substantially purified gene product polypeptide, related to a given gene, comprising:
selecting a vector for delivering cDNA having a nucleotide sequence corresponding substantially to the nucleotide sequence of said gene;
inserting said DNA into the vector;
introducing the vector into a cell culture to form an expression system for the propagation of said polypeptide;
permitting the cells to grow;
disrupting the cells;
extracting from the cells the polypeptide; and
purifying the polypeptide.

2. A method of claim 1, wherein said vector is a virus.

3. A method of claim 1, wherein said virus is a baculovirus.

4. A method of claim 1, wherein the virus is Autographa californica nuclear polyhedrosis virus.

5. A method of claim 1, wherein said cDNA has polyhedrin gene promoter characteristics.

6. A method of claim 1, wherein said cDNA has a promoter of a polyhedrin gene and said cloned cDNA is inserted downstream of said promoter.

7. A method of claim 1, wherein said cells are eukaryotic cells.

8. A method of claim 1, wherein said cells are spodoptera frugiperda cells.

9. A method of claim 1, wherein said purifying is accomplished by immunoaffinity chromatographic techniques.

10. A method of claim 1, wherein said cDNA is derived from an eukaryotic gene.

11. A method of claim 1, wherein said cDNA is derived from an eukaryotic cancer suppressor gene.

12. A method of claim 1, wherein said cDNA is derived from the retinoblastoma gene.

13. A polypeptide expression system for expressing gene product polypeptides related to a given gene, comprising:
a vector for delivering and a cell culture.

14. An expression system of claim 13, wherein said vector is a virus.

15. An expression system of claim 13, wherein said vector is a baculovirus.

16. A system of claim 13, wherein said vector is Autographa california nuclear polyhedrosis virus.

17. A system of claim 13, wherein the cells of said cell culture are insect cells.

18. A system of claim 13, wherein said cells are spodoptera frugiperda cells.

19. A substantially purified polypeptide, related to a given gene, made by the method comprising:
selecting a vector for delivering cDNA having a nucleotide sequence corresponding substantially to the nucleotide sequence of said gene;
inserting said DNA into the vector;
introducing the vector into a cell culture to form an expression system for the propagation of said polypeptide;
permitting the cells to grow;
disrupting the cells;
extracting from the cells the polypeptide; and
purifying the polypeptide.

20. A polypeptide of claim 19 including deriving said cDNA from the retinoblastoma gene.

21. A method of treating a cell having a defective, absent or mutative cancer suppressor gene comprising:
identifying the defective, absent or mutative gene;
determining the intact counterpart of said gene where said counterpart is the naturally occurring gene or a clone thereof;
preparing the protein product of the counterpart gene;
delivering said protein product to the cell.

22. A method of claim 21 wherein said delivery includes microinjecting said protein product into the cell.

23. A method of claim 21 wherein said purifying including dialyzing said protein into a buffer.

24. A method of claim 21 wherein said buffer contains Tris-HCl, KCl, EDTA, DTT and glycerol.

25. A method of claim 24 wherein said Tris-HCl has a pH of 7.4.

26. A method of claim 23 wherein said buffer includes 1 part DTT, 1 part EDTA, 100 parts KCl and 200 parts Tris-HCl.

27. A method of claim 21 wherein said identifying includes determining the presence or absence of protein products and measuring the amount of protein produced.

28. A method of claim 21 wherein said measuring includes using an antibody specific for said protein to determine if an immunocomplex of said antibody and said protein if formed.

29. A pharmaceutical composition comprising a polypeptide as the active ingredient and a physiologically suitable carrier.

30. A pharmaceutical composition of claims 29 wherein said polypeptide is pp110^{RB}.

31. A pharmaceutical composition of claim 29 wherein the active ingredient is selected from the protein products of breast cancer suppressing genes, Wilm's tumor suppressing genes, Beckwith-Wiedemann syndrome suppressing genes, transitional cell carcinoma suppressing genes, neuroblastoma suppressing genes, small cell lung carcinoma suppressing genes, renal cell carcinoma suppressing genes and colorectal carcinoma suppressing genes.

32. A pharmaceutical composition of claim 29 wherein the active ingredient has the following amino acid sequence:
